# EUROPEAN PATENT SPECIFICATION

(11) **EP 1 325 335 B1**
(45) Date of publication and mention of the grant of the patent: **12.04.2006**
(21) Application number: 01982391.3
(22) Date of filing: 03.10.2001
(51) Int. Cl.: G01N 33/566, A61P 25/28

(54) **METHODS FOR DIAGNOSING AND TREATING HUNTINGTON'S DISEASE**
VERFAHREN ZUR DIAGNOSE UND BEHANDLUNG VON HUNTINGTONSERKRANKUNG
METHODE DE DIAGNOSTIC DE LA MALADIE DE HUNTINGTON ET MOYENS DE TRAITEMENT DE CETTE MALADIE

(30) Priority: 03.10.2000 IT MI20002137
(43) Date of publication of application: 09.07.2003
(73) Proprietor: UNIVERSITA'DEGLI STUDI DI MILANO, I-20122 Milan (IT); Universita' Degli Studi di Ferrara, 44100 Ferrara (IT)
(72) Inventor: CATTABENI, Flaminio, Nicola, I-20133 Milan (IT); CATTANEO, Elena, I-20047 Brugherio (IT); ABBRACCHIO, Mariapia, I-20146 Milan (IT); VARANI, Katia, I-44038 Pontelagoscuro (IT); BOREA, Pier, Andrea, I-44100 Ferrara (IT)
(74) Representative: Gervasi, Gemma
(86) International application number: PCT/EP2001/011425
(87) International publication number: WO 2002/029408

(56) References cited:
- WO-A-00/13681
- WO-A-00/13682
- GLASS M ET AL: "The pattern of neurodegeneration in Huntington's disease: A comparative study of cannabinoid, dopamine, adenosine and GABAA receptor alterations in the human basal ganglia in Huntington's disease." NEUROSCIENCE, vol. 97, no. 3, 2000, pages 505-519, XP002211713 ISSN: 0306-4522
- PIERCE K D ET AL: "MOLECULAR CLONING AND EXPRESSION OF AN ADENOSINE A2B RECEPTOR FROM HUMAN BRAIN" BIOCHEMICAL AND BIOPHYSICAL RESEARCH COMMUNICATIONS, vol. 187, no. 1, 1992, pages 86-93, XP002211714 ISSN: 0006-291X
- DATABASE BIOSIS [Online] BIOSCIENCES INFORMATION SERVICE, PHILADELPHIA, PA, US; May 1998 (1998-05) MUNRO ROBIN ET AL: "Differential expression of adenosine A2A and A2B receptor subtypes on myeloid U937 and THP-1 cells: Adenosine A2B receptor activation selectively stimulates cAMP formation and inhibition of TNFalpha release in THP-1 cells." Database accession no. PREV199800428716 XP002211715 & DRUG DEVELOPMENT RESEARCH, vol. 44, no. 1, May 1998 (1998-05), pages 41-47, ISSN: 0272-4391
- BARALDI P G ET AL: "Design, synthesis, and biological evaluation of a second generation of pyrazolo(4,3-e)-1,2,4-triazolo(1,5-c)pyrim idines as potent and selective A2A adenosone receptor antagonists" JOURNAL OF MEDICINAL CHEMISTRY, AMERICAN CHEMICAL SOCIETY. WASHINGTON, US, vol. 41, 1998, pages 2126-2133, XP002163351 ISSN: 0022-2623

## Description

### FIELD OF THE INVENTION

The present invention relates to the field of therapy for diseases of the central nervous system. The invention concerns an *in-vitro* method for early diagnosis of the onset of Huntington's Disease based on the assessment of abnormal adenylate cyclase activity on the increase of A_{2A} receptor and in the use of drugs that inhibit this activity, for the prevention and/or treatment of this disease.

### PRIOR ART TECHNIQUES

Huntington's Disease (HD) is an hereditary disorder of the neurodegenerative type, highly debilitating from the motor and psychiatric point of view (Hayden MR Huntington's chorea, Springer-Verlag:London, Berlin, Heidelberg. 1981). In most cases onset occurs in the fertile age (around 35) with an incidence of one case in 10,000 and a mean duration of the disease of about 17 years. This disease inevitably leads to death after a long devastating clinical course characterized by progressive deterioration and irreversible disability. Often immediately aware of their situation and future, even before evident behavioural changes, people affected by HD tend to isolate themselves and discontinue any type of working and social relationships. Besides the consequences for the patient and his/her family, the long clinical course means that this pathology has extremely high economic costs for society.

There is no effective therapeutic treatment for HD; the choreform movements typical of this disease can be reduced, only in part, by anti-psychotics or reserpine (Merck Manual, Ed. Merck Res.Laboratories, 17^{th} ed., 1999, 1464).

The neuropathological damage inherent in HD comprises degeneration of the neurons of the basal ganglia, cerebral areas in charge of controlling involuntary movements (Reiner A., et al, Proc. Natl. Acad. Sci. **85**, 5733-37, 1988.).

The genic defect responsible for the disease was identified in 1993 (Huntington's Disease Collaborative Research Group) and consists in an expansion of the CAG triplet coding for the amino acid glutamine, at the level of the N-terminal end of a protein know as huntingtin. In healthy subjects, this triplet has a maximum number of repetitions of 36 units; however in those affected, there is an increase in these repetitions ranging from 38 to 120 units. Within the scope of this variability, it has been observed that the greater the number of repetitions, the earlier the onset of the disease is (with a view to this see also below), which is in any case present in 100% of subjects with mutation and is transmitted with dominant autosomic characteristics (just one mutant allele is sufficient to evoke the pathology, Brinkman et al., Am J Hum Genet. **60**, 1202-1210, 1997. et al., 1997). Although it allows identification of persons who will develop the disease, the genetic test does not guarantee absolute prediction of the age of onset. In fact, for the most common range of CAG, which varies from 45 to 50 CAG, the age of onset may vary by up to 20 years. When the CAG increase (over 60) correlation becomes narrower, while with CAG below 42 the possible spectrum of onset of the disease may be between 30 and 90 years old. Therefore, a test that allows to monitor the progression of the disease may be extremely useful for the purpose of timely pharmacological action.

Recent studies have suggested that a "gain-of-function" mechanism is involved in the etio-pathogenesis of the disease due to the expansion of CAG in the huntingtin molecule; this phenomenon leads to cellular synthesis of a mutant huntingtin. The huntingtin has a physiological function as an anti-apoptotic molecule (Rigamonti et al. J. Neurosci. 20(10), 3705-3713, 2000). On the basis of this evidence, it was proposed that the presence of CAG expansion could also cause "loss-of-protective- function" of the huntingtin and that this contributes to the onset and/or progression of the pathology.

A typical characteristic of the basal ganglia neurons subject to degeneration in HD is the high density selective expression of a specific subtype of receptor for adenosine, the adenosine A_{2A} receptor (Fredholm et al. Pharmacol. Rev. **46,** 143-156, 1994). This receptor's role in regulating motor activity at the level of basal ganglia has been known for some time (Ferrè et al., S, Neurosci. 1992, **51**, 5402-5406). Its importance also in cell vitality was suggested recently, although data in the relevant literature are somewhat contrasting (to review, see: Abbracchio & Cattabeni, Annals New York Acad. Sci., (1999), 890: 79-92.). For example, anti-ischaemic effects were described as a result of activation of the A_{2A} receptor present on the artery wall, on circulating platelets and neutrophils (see Abbracchio & Cattabeni, 1999, supra); in some experimental models of convulsions, stimulation of the A_{2A} receptor via the selective agonist 2-hexinyl-NECA (Adami et al., Eur. J. Pharm. (1995) 294: 383-389) or other adenosine agonists (Klitgaard et al., Eur. J. Pharmacol. (1993), **242**: 221-228) markedly reduces convulsions induced by pentyl-tetrazole or β-carboline respectively; similarly, A_{2A} agonists proved to be neuroprotective in an experimental model of global ischaemia (Kulinski et al., Drug Dev. Res. (2000) **50**:70.).

However, these data are in contrast with the results of other studies, that show that rather than through stimulation, anti-ischaemic protective effects can be obtained through blocking the actions of the A_{2A}. receptor. Inactivation of the striatal A_{2A} receptor via knockout (Chen et al., Drug Dev. Res; (2000) **50**: 71) or by blocking it with selective antagonists (Monopoli et al., Drug Dev. Res. (2000) **50**:70; Morelli et al., Drug Dev. Res. (2000), **50**:18) in fact led to beneficial effects in experimental models of Parkinson's disease. In an experimental model of focal ischaemia, the A_{2A}-selective antagonist SCH 58261 proved to be capable of significantly reducing the cortical damage associated with occlusion of the middle cerebral artery (Monopoli et al., NeuroReport, (1998), **9**, 3955-3959. A study on HD, performed on an experimental chemical model of neurotoxicity (inducing neuron death through intra-striatal injection of quinoline acid), showed some beneficial effects after the administration of agonists of the A_{2A} receptor (Popoli et al., European Journal of Pharmacology, (1994), **25**:5-6).

Experimental models of chemical lesion like the one mentioned above have important limitations: the first concerns their unspecificity, as they are used for the study of neurodegenerative diseases that are very different from one another as regards symptoms, etiology and response to treatments; a second limitation, particularly significant for HD, is due to the fact that neurodegeneration is obtained by poisoning cells with toxic principles (i.e. quinoline acid), a phenomenon that has nothing in common with the onset of HD in man, with a neuropathologic picture similar to ischaemic damage. In order to obviate these limitations genetic models of HD were recently developed: these are cell lines and/or transgenic animals containing the aforesaid mutation (expansion of the CAG triplet) in the huntingtin. Unlike the chemical models, the genetic models progress towards cell death following the actual pathological course of the disease and can thus provide much more accurate information on the possibility of actual treatment and prevention. In a recent study performed on a model of HD transgenic animal a decrease in adenosine A_{2A} receptors was found (summarised in: Cha, J.-H.J. Trends Neurosci. (2000), 23, 387-392). As already stressed, there are currently no specific therapies available, of either the preventive or curative type, for patients suffering from HD It is therefore obvious that any pharmacological approach that can lead to the development of drugs capable of delaying onset of the disease, reducing its seriousness and/or slowing down its progress represents a significant development of interest to the pharmaceutical company.

At the moment no studies that propose efficacious treatment strategies for HD have emerged. Therefore, there is still an enormous need for drugs to treat HD that are truly efficacious both at preventive and therapeutic level.

### SUMMARY

The present invention is based on the identification of abnormal behaviour of the A_{2A} receptor for adenosine and of its transduction system (adenylate cyclase enzyme) in cells genetically predisposed to develop HD and in circulating cells obtained from patients with this disease. This behaviour is made evident by an increase in the number of A_{2A} receptors in the cells of affected patients and in cells genetically predisposed to develop the disease, as well as by overproduction of cyclic AMP following stimulation with A_{2A} agonist compounds. The invention exploits this increase in the A_{2A} receptors and overproduction of cyclic AMP as diagnostic markers in an *in-vitro* method for early detection of the onset of HD and to monitor its progression. The invention also describes the use of compounds with A_{2A} antagonist action that, being capable of blocking this abnormal behaviour, prevent pathological progression of the cell and thus form a class of drugs useful for the treatment and prevention of this disease.

### DESCRIPTION OF THE FIGURES

**Figure 1**: RT-PCT for the A_{2A} receptor in parental striatal cells (ST14A), or in striatal cells expressing wild-type (wt) or mutant (mu) huntingtin, full-length (FL) or truncated (N548) (for the initials, see: Experimental part). PCR parameters: annealing at 54°C for 30 sec., extension at 72°C for 45 sec., 40 cycles.
**Figure 2**: Production of cyclic AMP induced by forskolin 3 µM in the parental clone (ST14A) (▲) and in the clone N548mu (■) in the presence of increasing concentrations of substrate (ATP, "X" axis):
   (2A) Michaelis-Menten analysis
   (2B) transformation of the experiment data according to Lineweaver-Burk
**Figure 3**: Production of cyclic AMP induced by NECA (0.1 nM-10 µM). Panel **A:** parental cells (ST14A) (◆), cells FLwt (V), and N548 wt (•);Panel B: ST14A(■), FLmu (▲), and N548 mu (•).
**Figure 4**: Induction of programmed cell death by serum deprivation in N548wt and N548mu cells. Cell death has been induced by shifting cultures to serum-free medium in the absence (black columns) or presence of the indicated A_{2A} receptor antagonists. Data are the mean ± S.E. of 6 experiments run in triplicate and are expressed as % of total cell death. *p < 0.05 vs. control, Scheffe's test.

### DETAILED DESCRIPTION OF THE INVENTION

The present invention is based on studies, performed by the applicants, on an experimental genetic model of HD and on platelets obtained from patients with HD. The experimental genetic model, which reflects the genetic abnormality found in patients affected by HD, is composed of neuronal cells, the genome of which contains the expansion of the CAG triplet; the cells thus modified express, analogously to the *in-vivo* pathological situation, the mutant huntingtin protein.

Working on this experimental model, the applicants unexpectedly observed that:
(i) the presence of mutant huntingtin in the neuronal cells affected by HD induces abnormal behaviour of the adenyl cyclase system responsible for cyclic AMP synthesis; this behaviour is expressed as overproduction of cyclic AMP induced by stimulation with A_{2A} agonist compounds;
(ii) the abnormal behaviour of the adenyl cyclase system, as directly correlated to the cells affected by HD, can be used as a diagnostic marker to monitor the onset and progression of HD;
(iii) the abnormal behaviour of the adenyl cyclase system is responsible for onset of the symptoms of HD;
(iv) the abnormal behaviour of the adenyl cyclase system is efficaciously inhibited by compounds with A_{2A} antagonist action; these can therefore be used in the prevention and treatment of HD;
(v) the aforesaid abnormal behaviour of the adenyl cyclase system is part of a general abnormality (aberrant amplfication) of the adenyl cyclase A_{2A} receptor transduction system, which was also confirmed by the Applicants in patients suffering from HD, in whom the aberration also becomes visible through an increase in the density of the A_{2A} receptors on circulating blood cells; this parameter may thus also be used as a diagnostic marker to monitor the onset and progression of HD;

These deductions are based on results set down in detail in the experimental part.

On the basis of these observations, the present invention is aimed at a method for the diagnosis of HD that utilizes, as diagnostic marker, an abnormal increase in the cellular production of cyclic AMP, said increase being induced by A_{2A} agonist compounds. The method is thus characterised by treating a sample of cells to be analysed with an A_{2A} agonist compound, followed by assessing the amount of cyclic AMP produced by the cells. The method is especial useful for monitoring the onset and/or progression of HD

The cells to be analysed are conveniently peripheral cells, preferably haematic cells such as platelets. These cells of haematic origin are obtained from whole blood taken from the patient and kept vital in appropriate cell culture systems using techniques know to the state of the art in this field; a method for recovering cells and keeping them in vital conditions is described in the article Varani et al., Circulation, 102(3):285-9, 2000. The adenylate cyclase activity can be determined with techniques known in the art, such as with the procedure below:
a- assessing, on healthy reference cells, the increase in cellular production of cyclic AMP caused by treatment of those cells with an A_{2A} agonist compound
b- repeating step a. on the cells to be analysed
c- comparing the value obtained in step a., with the one obtained in b.

It has been found by the Applicant that cells affected by HD show, with respect to healthy cells, an increased response to the stimulus with A_{2A} agonist, which is made evident by producing higher amounts of cyclic AMP. In case of full-blown HD, the production of cyclic AMP is increased by about 20% or more, with respect to healthy cells.

The test is preferably performed at physiological pH (7.4), and keeping the temperature between 30° and 38°C, i.e. at 33°C.

In steps a. and b. the presence of cyclic AMP can be quantified using known techniques, such as colorimetric, enzymatic, electrochemical systems, etc.; a method for determining cyclic AMP is described in Br.J.Pharmacol., 122(2)386-392 (1997).

In step b., one or more compounds with A_{2A} agonist action can be used. Preferably these compounds are capable to stimulate adenylate cyclase with concentrations efficacious at 50% (EC₅₀) in the range from 10⁻¹⁰ M to 10⁻⁴ M. These compounds are the agonist N-ethylcarboxamido-adenosine (NECA) and its derivatives such as the compounds described in Klitgaard et al., Eur. J. Pharmacol. (1993), **242**: 221-228, or by Cristalli et al., J. Med. Chem. (1992) 35: 2363-2368.

The A_{2A} agonist can be added, in case of samples consisting of cell lines, by simply adding the compound to the cell culture media; in the case of cell homogenates obtained from peripheral cells of patients, by adding the compound to the reaction buffer used to test adenyl cyclase activity.

The optimum quantity of agonist to be added can be assessed case by case via appropriate calibration tests; in this study efficacious responses of the adenyl cyclase system were detected with 100-300 nM concentrations of A_{2A} agonist. A_{2A} agonists at high concentrations (µM) are also used to verify the block of the agonist's effect.

A further embodiment of the present invention is a method for the diagnosis of HD on a cells' sample, characterised by assessing the receptor density of A_{2A} receptors in said cells, with respect to reference cells not affected by HD

This method, utilising as a diagnostic marker the increase of A_{2A} receptor density in cells affected by HD, does not require treatment of the cells with A_{2A} agonsits.

The receptorial density of adenosine A_{2A} receptors (also indicated as Bmax) can be determined with techniques known to the art, such as in Varani et al., Circulation, 102(3), 2000, 285-9. Determination can, for example, be performed using the following method:
a. assessment of the total binding obtained incubating a fraction of the platelet preparation (i.e. 100 µg of sample/protein) with a radioligand of the A_{2A}. receptor such as 3H-ZM 241385 used at a concentration saturating all the A_{2A}. receptors (i.e. for ³H-ZM 241385: 5 nM). The use of a saturating concentration allows an immediate measurement of the maximum number of A_{2A}. receptors present in the platelet membranes.
b. assessment of the non-specific binding of the same radioligand obtained with the same quantity of platelet preparation of the patient and the same radioligand concentration, and of an excess of non-radioactive ligand (i.e. ZM 241385 at a concentration of 1 µM) to totally eliminate the specific receptorial bond.
c. calculation of the specific binding as the difference between the two measurements performed in points a. and b. The value of the specific binding indicates the density of the receptors or Bmax expressed as fmoles of receptor / mg of protein or million of cells. This value is equivalent to the Bmax, generally expressed as fmoles of bonded ligand / mg of protein or millions of cells;
d. comparison of this difference to the one obtained from samples of human cells taken from healthy individuals tested in parallel.

The method of calculating the specific and non specific binding (steps a. and b.) is described in Varani et al., 2000, mentioned cited above; the test is generally performed in a buffer solution, physiological pH (7.4) and keeping the temperature between 30° and 38°C, i.e. at 33°C; the non-radioactive ligand used in step b. can be any compound capable of binding specifically to the A_{2A} receptor with Ki values of 10 nM-10 µM.

An example of an alternative method for assessing the A_{2A} receptorial density (Bmax) is the Scatchard analysis with radioligands for the A_{2A} receptor. This case contemplates the use of various concentrations of radioligand (usually 5) for each of which the total binding (a) and non-specific binding (b) value is determined. For each concentration of ligand the corresponding specific binding (c) value is then calculated. This type of analysis makes it possible to simultaneously assess the affinity of the binding (Kd) and the receptorial density (Bmax) (see Varani et al., 2000, cited above).

The receptorial density (Bmax) can also be determined using methods of non-radioactive detection of the number of A_{2A} receptors, such as methods using antibodies directed against the human A_{2A} receptor and similar techniques. For complete review of drug-receptor interaction and its theoretic presuppositions, of the radiochemical methods used to study this interaction and analysis of the results, see Kenakin, "Pharmacologic Analysis of Drug-Receptor Interaction", Lippincott-Raven Publ., 3rd ed., 1997.

In the present diagnostic method, cells of subjects with full-blown HD show significantly higher B-max values than those of healthy subjects; by significantly higher we intend at least 30%.

The usefulness of the diagnostic method described herein is especially evident in the case of HD in the subclinical state, when the disease is still not evident from macroscopic symptoms, or even in the case of patients who, although still healthy, are genetically predetermined to develop HD in the course of their life. In these cases the greater the increase in adenyl cyclase activity or receptorial density, the earlier the onset or progression of the disease will be. Therefore, the diagnostic methods claimed herein can be usefully applied to the field of preventive diagnosis, screening of patients carrying the mutant gene, as confirmation of neonatal diagnosis or as a diagnostic complement to define the state of progression of the disease in patients with controversial symptoms.

The possibility of early diagnosis of progression of the disease offered by the present method provides excellent conditions for efficacious curative treatment.

A further object of the present invention is a diagnostic kit for detection of the state of HD, composed of: (i) a substrate for maintaining a cells' sample in vital conditions (ii) an appropriate amount of an A_{2A} agonist compound, (iii) a system for the determination of cyclic AMP.

The substrate can be any system allowing to maintain the cells in vital conditions for at least the time necessary to perform the assessment test of cyclic AMP. Preferably, the substrate is a culture medium for cells, or a buffer for cell homogenates; these systems can be present in the kit in a ready-to-use form, o as an anhydrous powder to be diluted with water at the time of use.

The A_{2A} agonist (ii) can be preserved in the form of a solution, i.e. in a vial, or preserved in the anhydrous state, i.e. in sachets to be dissolved extemporaneously in the culture system / homogenization buffer at the time of performing the test.

The system to determine cyclic AMP (iii) can be of various types, i.e. colorimetric or radiochemical. In this case, the kit can be equipped with another container for the chromogenic reactive, or this reactive can be incorporated in the culture system (i). The quantities of cyclic AMP can be assessed with reference to a chromatic scale that can be included in the kit.

The kit can also be provided with mixing systems to facilitate dissolution of the agonist, and thermostatic means to keep the temperature of the culture system and/or incubation in optimum conditions during the test.

In the study on which the invention is based, the Applicants observed that cells affected by HD and which contain the underlying genetic change (expansion of the triplet CAG), show overactivity of the adenylate cyclase enzyme, that can be made evident as an over-production of cyclic AMP after stimulation with an A_{2A} agonist compound; this overactivity (here identified as "abnormal behaviour") is typical of cells containing the underlying genetic alteration causing HD, while it is not found in healthy cells.

Therefore, a further object of the present invention comprises the use of antagonist compounds of the adenosine A_{2A} receptor for the preparation of a drug useful in the prevention and/or treatment of HD.

Preferably, the A_{2A} antagonist compound blocks the effects of adenosine on the A_{2A} receptors at concentrations (constants of affinity, Ki) ranging from 10⁻¹⁰ to 10⁻⁴ M. The measurement of the Kᵢ can be made using techniques known to the art, as described in Varani et al., Br. J. Pharmacol. 122:386-392, 1997.

A_{2A} antagonists that may be used for the purposes of the present invention are the compounds:
5-amino-7-(2-phenylethyl)-2-(2-furyl)pyrazolo[4,3-e]-1,2,4-triazolo[1,5-c]pyrimidine, (SCH 58261) and derivatives thereof;
4-[2-[[7-amino-2-(2-furyl)[1,2,4]triazolo[2,3-a][1,3,5]triazin-5-yl]amino]ethyl]phenol, (ZM 241385) and derivatives thereof;
(7-amino-2(2-furyl)-5(2-(4-hydroxyphenyl)ethyl)amino(1,2,4)triazolo(1,5-a)(1,3,5)triazine and derivatives thereof;
5-amino-8-(4-fluorobenzyl)-2-(2-furyl)pyrazolo[4,3-e]-1,2,4-triazolo[1,5-c]pyrimidine, (8FB-PTP) and derivatives thereof;
5-amino-7-(4-fluorobenzyl)-2-(2-furyl)pyrazolo[4,3-e]-1,2,4-triazolo[1,5-c]pyrimidine, (7FB-PTP) and derivatives thereof;
8-styrylxanthines, such as 8-(3-isothiocyanatestyryl)-caffeine, 8-(3-chlorostyrylcaffeine), (CFC) and derivatives thereof;
(E/Z)-7-methyl-8-(3,4-dimethoxystyryl)xanthine, (KF 17837) and derivatives thereof;
5-amino-9-chloro-2-(2-furyl)-1,2,4-triazolo[1,5-c]quinazoline, (CGS 15943) and derivatives thereof;
caffeine and xanthine derivatives in general;
further A_{2A} antagonist compounds are described, for example, in J.Med.Chem., 1998, 41, 2126-33.

The inhibition of the abnormal adenyl cyclase behaviour reduces in particular some specific effects of HD directly linked to overactivity of the adenyl cyclase receptors of striatal neurons. Inhibition of adenyl cyclase overactivity thus in particular consents an increase in the survival of striatal neurons as determined by non-invasive cerebral analysis techniques; improvement of choreic movements associated with the pathology; improvement of the depressive state associated with the disease and the capacity for social interaction. Therefore, the proposed use is more specifically directed at treating the aforesaid conditions caused by HD.

For the purposes of the aforesaid uses, the A_{2A} antagonist compound can be administered in any available way, i.e. orally, intramuscularly, intravenously, transdermically, etc..

The A_{2A} antagonist compound can be formulated in all known forms of administration compatible with the active principle in question, such as tablets, capsules, microcapsules, solutions, suspensions, creams to be applied transdermically, formulations for inhalation, etc..

To sum up, the present invention establishes an important therapeutic contribution in the field of treatment and prevention of HD, offering:
(i) a method for verifying the presence of a receptorial aberration specifically associated with the genetic defect responsible for HD; this method allows monitoring of the state of progress of the disease in biological samples (cells) taken from the patient, from birth in the absence of symptoms, thus making it possible to implement preventive pharmacological operations targeted at delaying onset and reducing morbidity.
(ii) the necessary treatment of HD targeted at eliminating the aforesaid abnormal behaviour, by administration of selective A_{2A} antagonists.

The association of these two aspects of the invention allows therapy to be commenced early in subjects carrying the genetic defect, even in the absence of clinical symptoms to prevent, for the entire duration of the life of the person, onset of the debilitating symptoms of HD; this is an important step forward in the field of treating this disease.

The present invention further comprises a method for the diagnosis of neurodegenerative diseases caused by genetic mutations characterised by increased repetitions of the CAG triplet; this method is characterised by using, as a diagnostic markers either (i) the increase in cellular production of cyclic AMP following to treatment of said cells with A_{2A} agonist compounds, or (ii) the increase of receptor density of A_{2A} receptors, said increases according to (i) or (ii) being assessed on cells' samples, with respect to corresponding reference cells not affected by said neurodegenerative disease. The present invention further comprises the use of A_{2A} antagonist compounds for the treatment and/or prevention of genetic mutations characterised by increased repetitions of the CAG triplet.

The invention is now described by means of the following non-limiting examples. **EXPERIMENTAL PART**

### Example 1

### Assessment of the A_{2A}/adenyl cyclase receptor system in neuron cells genetically predisposed to develop HD

### Experimental model used

An immortalized cell line obtained from rat basal ganglia was used (parental cells, hereinafter indicated as ST14A) (Cattaneo et al.. Dev. Brain Res. 83, 197-208, 1994; 1996; Lundberg et al. Expe. Neurol. 145, 342-360, 1997; ; Cattaneo et al.

J.Neurosci. Research. 53, 223-234, 1998; ; Benedetti et al. Nat Med. 6, 447-450, 2000), from which some of the proposers previously obtained engineered sub-clones expressing Wild-type (wt) or mutant (mu) huntingtin, in the full-length (FL=Full-Length) or truncated (N548) form (Rigamonti et al., 2000, supra).

In the latter case, cDNA encoding only for the portion of the protein corresponding to the first 548 N-terminal amino acids was used.

In particular, the sub-clones used in the study were:
- **FLwt ("Full Length Wild-type")**: ST14A cells engineered with the cDNA encoding for full length wild-type huntingtin (3144 amino acids) with 23 CAG repetitions
- **N548wt ("N548 wild-type")**: ST14A cells engineered with the cDNA encoding for the first 548 amino acids of the wild-type huntingtin with 15 CAG repetitions
- **Flmu ("Full Length mutant huntingtin"):** ST14A cells engineered with the cDNA encoding for full length mutant huntingtin with 82 CAG repetitions
- **N548mu ("N548 mutant"):** ST14A cells engineered with the cDNA encoding for the first 548 amino acids of the mutant huntingtin with 120 CAG repetitions

Truncated huntingtins were chosen at the amino acid 548, as this represents the potential "cutting" site by caspases, protease enzymes that are believed to regulate *in vivo* the functionality of huntingtin through this mechanism (Wellington et al., J.Biol.Chem. **275**:19831-838, 2000).

Results obtained by the applicants show that the engineered sub-dones express the various huntingtins in a constant manner (Rigamonti et al., 2000, supra) and represent a good *in vitro* model to study the function of wild-type huntingtin and biochemical and molecular mechanisms at the base of the pathogeneticity of the mutant huntingtin.

### Methods used

### Cell cultures

The ST14A cells and clones derived from these are kept in DMEM with the addition of 10% serum (DMEM Sigma #D5671, Na Piruvato 0.11 g/l, L-glutamine 2mM Sigma#G7513, Pen-Strep Gibco #600-5075AE, FCS after decomplementation for 1 h at 56°C and sterilizing filtration).

Cells are kept incubating in culture plates at 33°C with 5% CO2. Cells are propagated when they reach 90% of confluence (Cattaneo et al., 1994; 1996; Cattaneo & Conti, 1998, supra).

### Extraction of total RNA.

Total RNA of ST14A cells, either parental or transfected with wild-type or mutant Huntingtin was extracted with TRIzol Reagent (Life Technologies). In short, the confluent cells in 100 mm diameter plates were washed with PBS, they lysated on a plate with 2 ml of TRizol Reagent. After incubation for 5 minutes at room temperature, the lysates were extracted with 0.4 ml of chloroform. The aqueous phase containing the RNA was separated via centrifugation at 12,000 x g for 15 min. and the total RNA was precipitated with 1 ml of isopropanol. After centrifugation at 12,000 g for 10 min the RNA pellet was washed with ethanol 70%, resuspended in DEPC-H2O and quantized with spectrophotometry.

### Reverse-Transcription Polymerase Chain reaction (RT-PCR).

Reverse-transcriptase was performed on 5 µg of total RNA according to the following protocol. The RNA was pre-incubated at 70°C for 10 min. with 250 ng of random primers in a total volume of 12 µl. The samples were then cooled quickly in ice for 2 min and 8 µl of the reverse-transcription mixture was added to each of them ("1st strand buffer"- Life Technologies - DTT 10 mM, dNTPs 0.5 mM each, 20 U of Ribonudease Inhibitor -RNAseOUT, Life Technologies -, 200 U of SuperScript II - Life Technologies). Reverse transcription was performed at 42°C for 1 h. At the end of incubation the reverse transcriptase was denatured at 70°C for 15 min.

For PCR amplification 2 µl (0.5 µg of cDNA) of the reverse transcription reaction were used. PCR was performed in a total reaction volume of 50 µl using MgCl₂ 1.5 mM, dNTPs 0.2 mM and 0.5 U of DyNAzyme EXT (Finnzymes). The A_{2A} receptor messenger was amplified using the following primers:
A2A Fw (5'-TGTCCTGGTCCTCACGCAGAG-3') and
A2A Rev (5'-CGGATCCTGTAGGCGTAGATGAAGG-3'), at a concentration of 0.25 µM each.

Simultaneously, an aliquot (of cDNA (0.5 µg) was used to amplify the messenger of the GAPDH as internal control, with the pair of primers GAPDH Fw (5'-TCGATGACAACTTTGGCATCGTGG-3') and GAPDH Rev (5'-GTTGCTGTTGAAGTCACAGGAGAC-3') at a concentration of 0.25 µM.

The PCR reaction was conducted according to the following protocol:

Initial denaturation at 95°C for 5 min., hybridation at 54°C for 30 sec., elongation at 72°C for 1 min., for a total of 40 cycles (30 cycles if the messenger for GAPDH is amplified), final extension at 72°C for 7 min.

The PCR products were separated with agarose gel at 1% in a TAE buffer and shown up by colouring with ethydium bromide.

### Binding measurement of the A_{2A} receptor

The cells are washed in PBS and detached with a cold hypotonic buffer (5 mM Tris HCl, 2 mM EDTA, pH 7.4). The cell suspension is homogenized with a specific "Polytron" instrument and subjected to centrifugation at 48,000 g for 30 min. The pellet is resuspended in a buffer containing 50 mM Tris HCl, 120 mM NaCl, 5 mM KCl, 10 mM MgCl₂ and 2 mM, CaCl₂ pH 7.4, incubated at 37°C for 30 min. with adenosine deaminase and again subjected to centrifugation at 48,000 g for 30 min. The resulting pellet is appropriately resuspended to obtain a concentration of 100-150 µg of protein per 100 µl and is used in the "receptor binding" experiments. In the saturation studies the membranes are incubated for 60 min. at 4°C with 8-10 different concentrations of radioligand ([3H]-ZM 241385) ranging from 0.05 and 10 nM. The unspecific binding is determined as binding in the presence of NECA 10 µM. Free and bonded radioactivity are separated by fast vacuum filtration using Whatman GF/B glass fibre filters with a specific filtration instrument (i.e. Brandel 48). The radioactivity withheld by the filters is then counted using a spectrometer (i.e. Beckman LS-1800) with an efficiency of 55-60%.

### (Preparation of cells for measurement of cAMP levels) italics

Cells are washed with PBS and detached from the flasks with a solution of PBS and 0.5% of tripsine. They are then resuspended in the culture medium and subjected to centrifugation for 10 min. at 200x g. The pellet is resuspended in the buffer composed of 120 mM NaCl, 5 mM KCl, 0.37 mM NaH₂PO₄, 10 mM MgCl₂, 2 mM CaCl₂, 5 g/L D-glucose, 10 mM Hepes-NaOH, pH 7.4 and again subjected to centrifugation for 10 min. at 200x g. The appropriately diluted cells (4 x10⁵ cells/test tube) are used in the experiments to measure cAMP levels.

### Measurement of cAMP levels

The cells are resuspended in the aforesaid buffer containing 2 U.I of adenosine deaminase and pre-incubated for 10 min. at 37°C. Forskolin 1 µM and increasing concentrations of N-ethyl-carboxyamide-adenosine (NECA, 1 nM-10 µM) are then added. The potency of the selective A_{2A} antagonists is determined by assessing the inhibition capacity of the levels of cAMP stimulated by NECA 100 nM. At the end of the reaction a solution of trichloroacetic acid (TCA) at 6% is added. The TCA suspension is subjected to centrifugation at 2000x g for 10 min. at 4°C and the supernatant is transferred to specific extraction test tubes with water saturated ether. The final aqueous solution is tested to measure cAMP levels using the method by Varani et al., 1997. The test tubes in which the test is performed contain in a final volume of 350 µl, 100 µl of sample, 125 µl of buffer composed of trizma base 100 µM, 2-mereaptoethanol 6 mM, amminophylline 8 mM, pH 7.4, 25 µl of [³H]-cAMP (corresponding to about 20,000 cpm) and 100 µl of "binding protein" binding cAMP, obtained in the laboratory from bovine surrenal capsules.

The dosage is completed by performing a calibration curve in which the following components are present:
a) known quantities of unmarked cAMP;
b) a white not containing the "binding protein";
c) standards respectively containing 0, 1, 2, 4, 7,10 pmoles of unmarked cAMP.

After mixing, samples are incubated for 90 min. at 4°C. Competition between marked and cold cAMP for the bond with the protein is blocked by adding 100 µl of an active carbon suspension at 10% to the aforesaid buffer containing bovine albumin 2%. The samples are then subjected to centrifugation at 2,000x g for 10 min. and 200 µl of the supernatant are transferred to vials with scintillation liquid (Ready gel, Beckman). The corresponding concentration of cAMP is calculated for comparison with the calibration curve. The experiment data were processed with the PRISM - Graph Pad program.

### Measurement of adenylate cyclase activity

The membranes obtained from the cells being examined are resuspended in the aforesaid buffer containing GTP 5 µM, 2 U.I. of adenosine deaminase and incubated for another 10' min. Lastly, forskolin 3 µM and increasing concentrations of ATP (1 nM-1 mM) are added. The reaction is terminated by transferring the samples to water at 100°C for 2 min. The samples are then cooled at ambient temperature and subjected to centrifugation for 10 min at 2000x g. The supematant obtained is used to dose cAMP present using the aforesaid method (Varani et al., Br. J. Pharmacol. **122(2),** 386-392, 9997).

### Results

### Presence of the A_{2A} receptor in parental cells and in sub-clones expressing the various forms of huntingtin

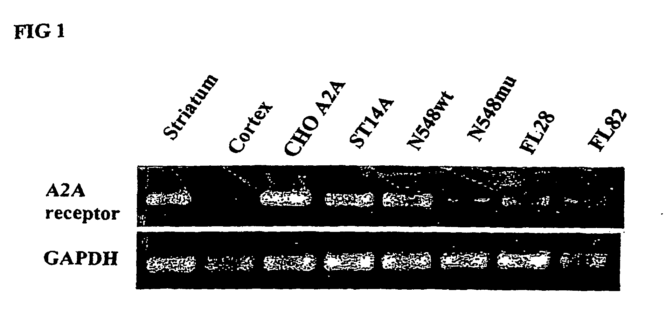

The experiment described in Fig. 1 shows an analysis of the mRNA levels for the *A*_{*2A*}. receptor obtained via RT-PCR, using as internal standard of amplification "primers" capable of recognizing the mRNA for a ubiquitously expressed gene such as GAPDH (see "Methods used" for greater details). The first three wells from the left (positive controls). indicate, respectively, the expression of the *A*_{*2A*} receptor in rat stratium (area belonging to the basal ganglia), in adult rat cortex, and in a clone of CHO cells that over-expresses this receptor (CHO A2A, Klotz et al., Naunyn-Schmiedeberg's Arch. Pharmacol. **360**, 103-108, 1999).

As expected, this analysis faithfully reproduces the expected levels of mRNA in the various samples analysed, with levels of expression considerably lower in the cerebral cortex (Fredholm et al., 1994, supra). Both the parental clone ST14A and the sub-clones expressing the various huntingtins (N548wt, N548mu, FLwt, Flmu) all transcribe the receptor, although at variable levels: in the case of clones expressing the mutant huntingtin, a reduction of Kd is observed (increase in the affinity of the binding), which is in line with the increase in the density of the *A*_{*2A*} receptors. (Table 1).

### Assessment of the responsiveness of the adenyl cyclase system

The adenosine *A*_{*2A*} receptor is a transmembrane receptor with seven domains connected functional to the G-protein of the sub-family Gs, stimulating the effector system of adenyl cyclase. In fact, activation of this receptor leads to an increase in the measurable levels of cyclic adenosine-monophosphate (cAMP, Fredholm et al., 1994, supra).

We studied the adenyl cyclase system in the various sub-clones analysing:
(i) the basal adenyl cyclase response;
(ii) the adenyl cyclase response to pharmacological agents that stimulate the production of cAMP in a receptor-independent manner;
(iii) the cyclase response to specific adenosynergic agonists;
(iv) the capacity of antagonist compounds of the *A*_{*2A*} receptor equipped with various chemical structures to block adenyl cyclase stimulation induced by adenosine agonists.

As shown in table 2, the basal adenyl cyclase response was no different in any of the engineered sub-clones than in the parental clone.

Exposure to forskolin 1µM, a direct activator of the enzyme, in the presence of an inhibitor of cAMP-dependent phosphodiesterases (RO 201724) causes overstimulation of cAMP production in the clones N548mu and Flmu (table 2). The experimental condition described above is used to show the responsiveness of the cAMP producing system, as a potent direct activator is used; this acts on the catalyst. sub-unit of the enzyme in the presence of an inhibitor of cAMP degradation. As shown in table 2, amplification of adenyl cyclase responsiveness is already evident even in the presence of forskolin alone in the clone N548mu.

These data suggest abnormal amplification of adenyl cyclase activity in clones expressing mutant huntingtin.

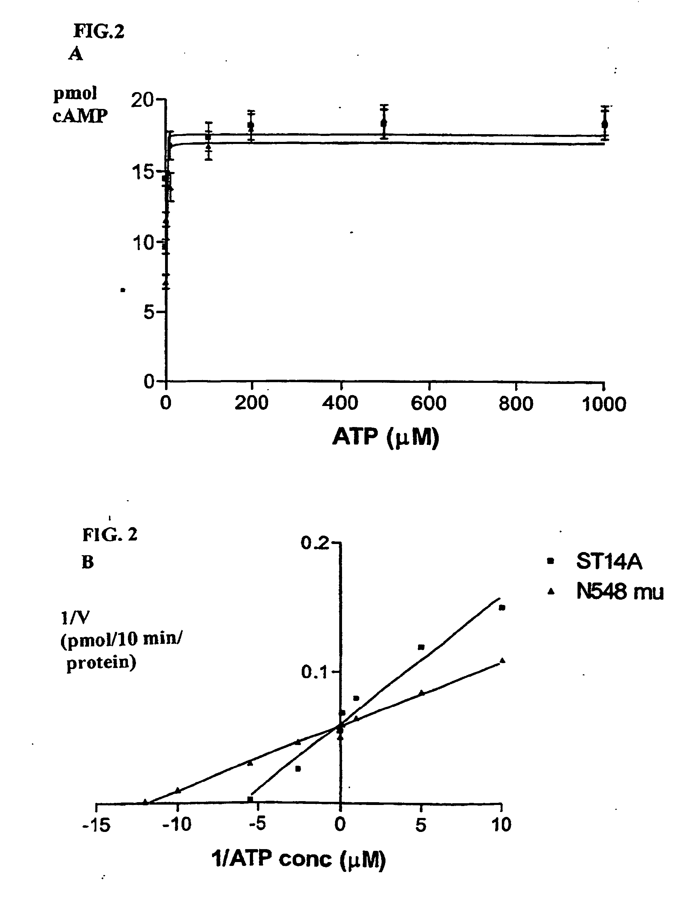

To define the nature of the alterations described above, we conducted Michaelis-Menten analysis, measuring the production of cAMP stimulated by forskolin in the parental clone (ST14A) and in the clone N548mu in the presence of increasing concentrations of substrate (ATP, indicated on the abscissa in the analysis described in figure 2). Both Michaelis-Menten analysis (Fig. 2A) and transformation of experimental data according to Lineweaver-Burk (Fig. 2B) indicate a significant reduction in the Km value of the enzyme in the clone N548mu with respect to the parental cells. The Km and Vmax values of the enzyme in these two clones are:

| | |
|---|---|
| *ST14A:* | *Vmax=16.8±0.4 pomli*/*min*. |
| | Km=180±4nM |
| N548mu: | Vmax=17.2±0.6 pomli/min. |
| | Km=82±3nM |

This result suggests that the presence of mutant huntingtin is associated to an increase in the affinity of the enzyme towards the substrate, as proved by the reduction in the Km value. Instead, there do not seem to be any alterations in the Vmax values of the enzyme in the clone N548mu with respect to the parental clone.

The abnormality found seems to be specific of the catalytic sub-unit of the enzyme, as the cAMP response induced by a direct stimulator of the G-proteins, such as a non-hydrolysable analogue of GTP (GTP-gammaS), was no different in engineered clones than in the parental clone (cAMP levels in the parental clone in the presence of 10 µM GTP-gammaS: 65±2 pmoles/10⁵ cells; clone N548mu: 64±1 pmoles/10⁵ cells). These data indicate that the hyperactivation of adenylate cyclase observed after stimulation of the A_{2A} receptor in cells expressing mutant huntingtin can also be regulated by alterations by the dimer Gβγ (Tang et al. Science **254:** 1500-3, 1991). This concept is set down in the next page.

### Assessment of the responsiveness to a denosine agonists

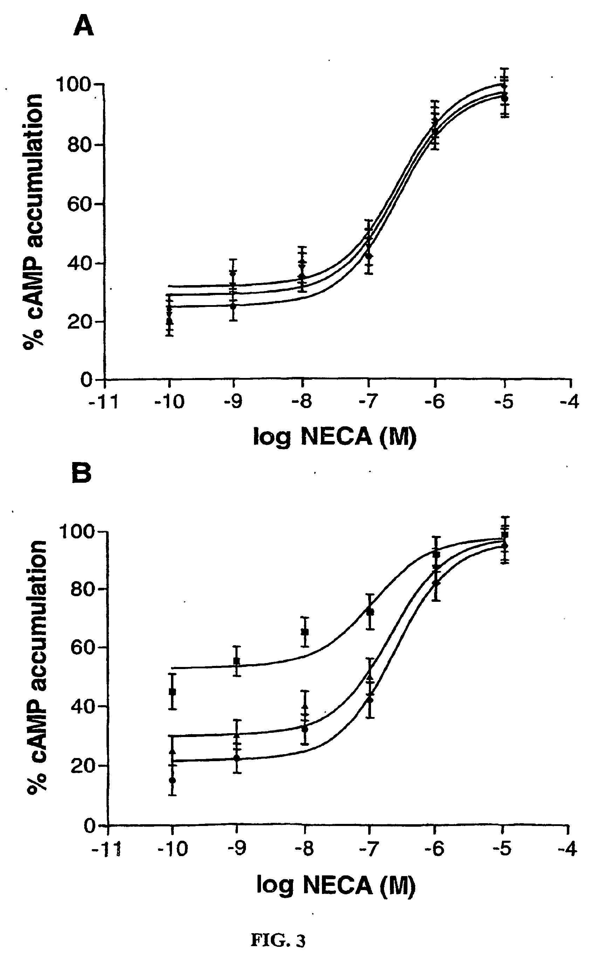

In addition to the alterations described above, engineered cells with mutant huntingtin also show irregular amplification of adenyl cyclase in response to adenosine agonists. In all clones the same adenosine NECA induced increases in concentration-dependent CAMP. Nonetheless; as shown in the: concentration-response curve indicated in Figure 3B, in the clones Flmu and N548mu, there are considerable increases in the production of cAMP with respect to the parental cells for almost all concentrations of agonist tested. However, no significant differences are found in the response to NECA at any of the concentrations tested in the clones FLwt e N548wt (Fig. 3A). To confirm the selectiveness of the aforesaid alterations, the EC₅₀ values for NECA are significantly lower than the controt values only in clones expressing wild-type huntingtin (Table 3). This result confirms that in the presence of mutant huntingtin, there is an increase not only in the activity of the catalytic sub-unit of adenylate cyclase as shown by previous data, but also in the responsiveness of the A_{2A} /adenylate cyclase receptor system. To confirm that NECA exercises its effects by activating the denosine receptors and not other receptors that may be co-expressed by these cells, we tested the capacity of various blockers of the A_{2A} receptor to antagonize the increases in NECA induced cAMP (table 4). CGS 15943, ZM 241385 and caffeine all proved to be capable of completely antagonizing the increases in cAMP induced by a concentration of NECA (100 nM) highly selective for the A_{2A} receptor. As further confirmation of the specificity of the effect, antagonism by caffeine proved to be concentration-dependent (table 4). Moreover, antagonism is evident both in the parental clone and in engineered clones, suggesting that the A_{2A} antagonists are capable of blocking abnormal amplification of the adenyl cyclase system present in cells expressing the mutant huntingtin.

**Table 1- Binding parameters of the ligand to the A_{2A} receptor [³H]-ZM 241385 in membranes of ST14A parental cells and in clones expressing the various forms of huntingtin**

| | **ST14A** | **FL wt** | **N548 wt** | **FL mu** | **N548 mu** |
|---|---|---|---|---|---|
| **Kd (nM)** | 2.30 ± 0.10 | 2.42 ± 0.08 | 2.48 ± 0.05 | 1.61 ± 0.06* | 1.32 ± 0.06** |
| **Bmax (fmoles/mg proteins)** | 92 ± 6 | 90 ± 8 | 90 ± 6 | 93 ± 5 | 90 ± 3 |

| | | | | | |
|---|---|---|---|---|---|
| • P <0.05 and ** P <0.01 with respect to the control, "t" Student test | | | | | |

**Table 2- Measurement of the basal cyclic AMP levels and those stimulated by forskolin in ST14A parental cells and in clones expressing wild-type or mutant huntingtin (full-length or truncated at the amino acid 548) (data are expressed in pmoles/10⁵ cells).**

| | **ST14A** | **FL wt** | **N548 wt** | **FL mu** | **N548 mu** |
|---|---|---|---|---|---|
| **Basal levels** | 18±2 | 19±2 | 18±1 | 20±2 | 20±1 |
| **Forskolin 1 µM** | 45 ± 5 | 54±6 | 47±3 | 55±5 | 72±4* |
| **Forskolin 1 µM + Ro201724 0.5 mM** | 58±4 | 60±6 | 63±6 | 78±7 | 88±5* |

| | | | | | |
|---|---|---|---|---|---|
| Statistical analysis was performed using the Student test; * P <0.01 versus the control. | | | | | |

**Table 3- Stimulation of the levels of cyclic AMP by NECA (1 nM-10 µM) in ST14A parental cells and in clones expressing wild-type or mutant huntingtin (full-length or truncated at the amino acid 548) The values of EC₅₀ (nM) are set down.**

| | **ST14A** | **FL wt** | **N548 wt** | **FL mu** | **N548 mu** |
|---|---|---|---|---|---|
| **EC**_{**50**} **(nM)** | **270 ± 10** | **253 ± 11** | **236 ± 13** | **198 ± 15*** | **93 ± 9**** |

| | | | | | |
|---|---|---|---|---|---|
| Statistical analysis was performed using the Student test | | | | | |
| • P <0.05 e ** P <0.01 vs control. | | | | | |

**Table 4- Effect of various antagonists of the A_{2A} receptor on stimulation of the production of NECA-induced cAMP in the ST14A parental cell and in clones expressing the various forms of huntingtin (data are expressed in pmole/10⁵ cells).**

| | **ST14A** | **FL wt** | **N548 wt** | **FL mu** | **N548 mu** |
|---|---|---|---|---|---|
| **Basal levels of cAMP** | 18 ± 2 | 19 ± 2 | 18 ± 1 | 20 ± 2 | 20 ± 1 |
| **Formation of NECA induced cAMP** | 38 ± 5 | 39 ± 5 | 40 ± 4 | 48 ± 3 | 55 ± 6 |
| **NECA + CGS15943 (1 µM)** | 19 ± 3* | - | - | - | 22 ± 4* |
| **NECA + ZM241385 (1 µM)** | 20 ± 4* | - | - | - | 21 ± 3* |
| **NECA + Caffeine (10 µM)** | 30 ± 3 | - | - | - | 36 ± 4 |
| **NECA + Caffeine (100 µM)** | 27 ± 3 | - | - | - | 34±3 |
| **NECA + Caffeine (1 mM)** | 24±3 | - | - | - | 26 ± 3 |

The formation of cAMP was measured in the absence of stimuli (basal levels) or in the presence of 100 nM NECA, in the absence or presence, as indicated, of various adenosine antagonists at the concentrations set down above. * P <0.01 with respect to the formation of cAMP stimulated by NECA, "t" Student test.

### Example 2

### Correlation between abnormal adenylcyclase activity and death of striatal cells in a genetic model of HD; cells survival in presence of A_{2A} antagonist compounds

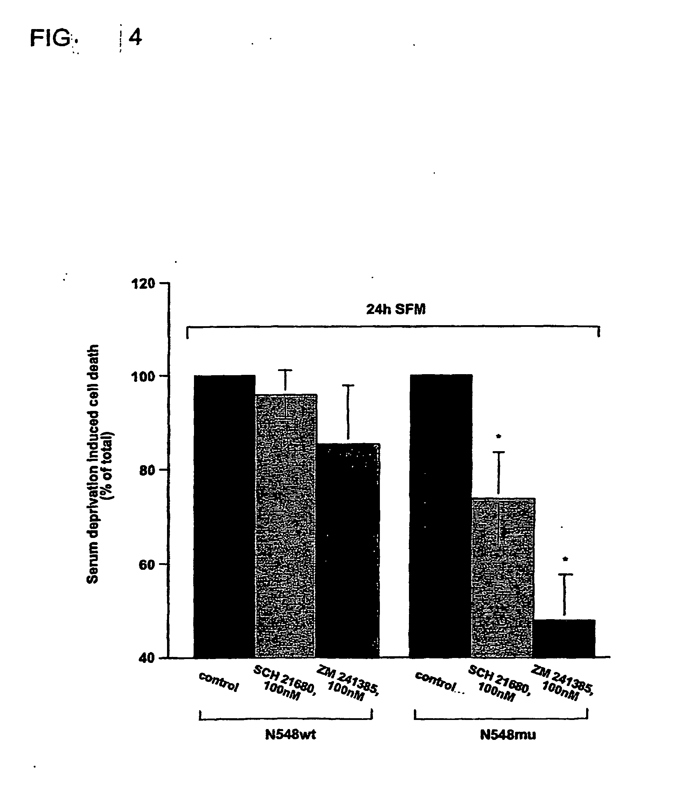

To assess the functional importance of the aberrant A_{2A} receptor phenotype and its possible correlation with susceptibility to cell death, we have performed experiments where the influence of A_{2A} receptor antagonists on the survival rate of ST14A cells have been specifically determined. ST14A cells are known to undergo programmed cell death upon serum deprivation (Rigamonti et al., J. Neurosci. 20(10), 3705-3713, 2000). We have assessed the ability of several A_{2A} receptor antagonists to counteract serum-deprivation induced cell death in ST14A cells engineered to express either wild-type (N548wt) or mutant Htt (N548mu) in its truncated form. Cells were grown in complete medium for 48 h, shifted to serum-free medium, and cell death determined by assessing mitochondrial function after further 24 h. A_{2A} receptor antagonists (either SCH58261 or ZM 241385, both utilized at a 100 nM concentration) have been added to cultures 24 h after beginning the experiment. As shown in Figure 4, in N548wt cells, the concomitant exposure of cells to either A_{2A} receptor antagonist did not significantly affect the extent of serum-deprivation induced cell death. In contrast, in N548mu cells, both A_{2A} receptor antagonists significantly reduced the extent of cell death associated to serum deprivation, being ZM 241385 more effective than SCH 58261. Interestingly, only in N548mu cells is adenylyl cyclase coupled to modulation of cell survival: in fact, blockade of A_{2A} receptors in cells expressing physiological Htt has little (if any) influence on the extent of serum-deprivation induced cell death. These data suggest that the aberrant increase of A_{2A} receptor-dependent adenylyl cyclase in cells expressing mutant Htt is associated to the anomalous activation of intracellular pathways coupled to induction of cell death.

### Example 3

### Assessment of the density of A_{2A} receptors (Bmax) on haematic cells of patients with HD.

### Test n° 1

### Experimental model

The status of the adenosine A_{2A}. receptor was verified on circulating cells (platelets) in patients with HD (10 heterozygote patients with diagnosis of full blown chorea), compared with healthy volunteers.

### Preparation of the cells and binding test

After being taken, the blood is conserved for a maximum of 6h at room temperature until separation of the platelets. The membranes are isolated from the platelets by centrifugation (Varani et al., 2000, op. cit.) and the A_{2A}.receptor is dosed via the radioligand ³H-ZM 241385 according to the binding procedure explained above (Varani et al., 2000). The platelet membranes of each subject were analysed according to Scatchard to allow calculation of a K_{d} value (nM, index of the affinity of the bond) and Bmax value (bound fmoles/mg protein, index of receptorial density).

### Results

Results showed that the Bmax value in patients affected by HD is about double the mean value found in the platelets of healthy subjects (P<0.01, see Table 5).

**Table 5 - Values of affinity and density of A_{2A} receptors in the membranes of human platelets of control subjects and heterozygote subjects with HD**

| Subjects | Kd | Bmax |
|---|---|---|
| | (nM) | (fmoles/mg proteins) |
| Control | 1.19 ± 0.22 | 128 ± 10 |
| n=10 | | |
| Mean age: 41±10 a | | |
| heterozygote HD | 3.05 ± 0.33* | 224 ± 7* |
| n=10 | | |
| Mean age: 38±9 a | | |

| | | |
|---|---|---|
| *, P<0.01 vs control subjects | | |

As can be seen in table 5, the Kd values were different in patients with HD compared with control subjects (P<0.01).

### Test n° 2

To verify whether the aberrant A_{2A} receptor phenotype could be utilized as a peripheral marker of HD, we have measured A_{2A} receptor binding and its coupling to adenylyl cyclase in 47 heterozygous and in 3 homozygous HD patients in comparison with control subjects.

After withdrawal from patients, blood is maintained at room temperature for up to 6 h, and separation of the various types of circulating cells is performed as previously described (Varani et al., Br. J. Pharmacol., 117, 1693-1701, 1996; Varani et al., Br. J. Pharmacol., 122, 386-392, 1997, Varani et al., Br. J. Pharmacol., 123, 1723-1731, 1998). After stabilization, blood is centrifuged to obtain platelet rich plasma (PRP). The PRP fraction is centrifuged several times to obtain washed platelets. To separate lymphocytes from monocytes and polymorphonuclear leukocytes blood is centrifuged on Ficoll-Hypaque density gradients. Several centrifugations and resuspensions in phosphate buffer are performed to obtain the lymphocytic fractions. The red pellet resulting from the above procedure and containing erythrocytes are supplemented with Dextran T500, the turbid upper layer containing leukocytes are removed and centrifuged to obtain neutrophil enriched cell suspension. Membranes are then prepared from isolated cells and A_{2A} receptor binding is performed with the A_{2A} receptor ligand 3H-ZM 241385 as previously described (Varani et al., Br. J. Pharmacol., 117, 1693-1701, 1996; Varani et al., Br. J. Pharmacol., 122, 386-392, 1997, Varani et al., Br. J. Pharmacol., 123, 1723-1731, 1998). Saturation binding experiments are performed by incubating membranes with 8 to 10 concentrations of the antagonist 3H-ZM 241385 in the range 0.01-10 nM. A Scatchard analysis leading to calculation of KD (nM, an affinity parameter) and Bmax (bound fmol/mg prot, an index of receptor density) has been obtained for each cell subpolation/patient.

**Table 6- Binding parameters of the A_{2A} adenosine receptor in human circulating blood cells of control and Huntington subjects**

| | **PLATELETS** | | **LYMPHOCYTES** | | **NEUTROPHILS** | |
|---|---|---|---|---|---|---|
| **Subjects** | **Kd (nM)** | **Bmax (fmol/mg protein)** | **Kd (nM)** | **Bmax (fmol/mg protein)** | **Kd (nM)** | **Bmax (fmol/mg protein)** |
| **Control** | 1.09±0.06 | 120±4 | 1.17±0.11 | 72±13 | 1.18±0.09 | 81±13 |
| | n=24 | n=24 | n=13 | n=13 | n=13 | n=13 |
| **HD** | 2.87±D.19 | 213±7 | 3.01±0.16 | 215±5 | 3.59±0.15 | 225±6 |
| **Heterozygous** | n=47 | n=47 | n=41 | n=41 | n=41 | n=41 |
| **HD** | 3.12±1.14 | 220±11 | 3.24±0.24 | 219±18 | 7.31±0.7 | 304±14 |
| **Homozygous** | n=3 | n=3 | n=3 | n=3 | n=3 | n=3 |

Table 6 shows alterations of both the affinity (KD) and density (Bmax) of A_{2A} receptors in both platelets, lymphocytes and neutrophils of HD patients. In particular, in all cell populations, there is an highly significant increase of the KD value (likely resulting from a malfunction of the receptor) and a marked increase of Bmax values, which are 2-4 fold higher with respect to control values. The aberrant increase of A_{2A} receptor function suggested by the in vitro studies in neural cells is hence also confirmed in the circulating cells of HD patients. The aberrant A_{2A} receptor phenotype is even more evident in HD homozygous subjects with respect to heterozygous subjects. Experimental data obtained in a transgenic HD animal model indeed suggest that normal Htt expression attenuates the phenotype induced by mutant Htt (Leavitt et al. Am J Hum Genet 68, 313 , 2001), suggesting that the severity (and prognosis) of the disease could be worse in homozygous HD subjects. Moreover, neutrophils seem to represent the circulating cell type where the aberrant A_{2A} receptor phenotype is maximally expressed.

Finally, we demonstrated that the alteration of A_{2A} receptor binding parameters in the peripheral cells of HD patients also correlates with an aberrantly increased coupling of this receptor to adenylyl cylase and cAMP formation.

Adenylyl cyclase assays has been performed as previously described (Varani et al., Br. J. Pharmacol., 117, 1693-1701, 1996; Varani et al., Br. J. Pharmacol., 122, 386-392, 1997, Varani et al., Br. J. Pharmacol., 123, 1723-1731, 1998). Adenylyl cyclase activity has been assayed by incubating blood peripheral cells in the absence (basal activity) or presence of 6-8 different concentrations of a typical adenosine agonist NECA. The reaction is terminated by the addiction of cold 6% trichloroacetic acid (TCA). The TCA suspension is centrifuged and the supernatant is extracted 4 times with water saturated diethylether. The final aqueous solution is tested for cyclic AMP levels by a competition protein binding assay.

**Table 7 - Potency of the A_{2A} adenosine receptor agonist NECA in increasing cAMP levels in human platelets, lymphocytes and neutrophils**

| | **PLATELETS** | **LYMPHOCYTES** | **NEUTROPHILS** |
|---|---|---|---|
| **Subjects** | **EC**_{**50**} | **EC**_{**50**} | **EC**_{**50**} |
| | **(nM)** | **(nM)** | **(nM)** |
| **CONTROL** | **308 ± 7** | **199 ± 4** | **168 ± 5** |
| n=10 | | | |
| **HD patients** | **119 ± 13*** | **79 ± 10*** | **85 ± 9*** |
| n=12 | | | |

| | | | |
|---|---|---|---|
| * P<0.01, Student's t test | | | |

As it can be seen in Table 7, in both platelets and neutrophils, the EC50 value of the A_{2A} agonist NECA (i.e., the NECA concentration necessary to yield an half-maximal stimulation of cAMP production) is significantly reduced in HD patients with respect to controls, indicating an increase of A_{2A} receptor-mediated cAMP formation.

Globally, all these data suggest that both the number of A_{2A} receptors (A_{2A} receptor Bmax values) and the EC50 values of A_{2A} receptor agonists obtained in circulating cells may be utilized as peripheral markers to monitor the development and progression of HD.

The above data on the receptorial density are in line with a generalized aberrant amplification of the adenyl cyclase A_{2A} receptor transduction system in the case of patients affected by HD. These data thus show the efficacy both of receptorial dosage and of measurement of adenyl cyclase activity as diagnostic markers to determine onset and progression of this disease.

## Claims

1. Method for diagnosis of Huntington's Disease on a cells.' sample *in-vitro* **characterised by** treating said cells with an A_{2A} agonist compound, followed by assessing the amount of cyclic AMP produced by said cells.

2. Method as claimed in claim 1, comprising the following steps:
a- assessing, on healthy reference cells, the increase in cellular production of cyclic AMP caused by treatment of those cells with a A_{2A} agonist compound;
b- repeating step a. on the cells to be analysed;
c- comparing the value obtained in step a., with the one obtained in b.

3. Method as claimed in claims 1 and 2, performed in a buffer solution, with a pH ranging from 6.5 to 8, at a temperature ranging from 30° to 38°C.

4. Method as claimed in claims 1 to 3, where the A_{2A} agonist used is N-ethylcarboxamido-adenosine (NECA)

5. Method as claimed in claims 1 to 4, where the A_{2A} agonist is used at a concentration ranging from 10nM to 300 nM.

6. Kit for the diagnosis of Huntington's Disease, composed of:
(i) a substrate for maintaining a cells' sample in viable conditions
(ii) an appropriate amount of an A_{2A} agonist compound
(iii) a system for the determination of cyclic AMP.

7. Kit as claimed in claim 6, where said substrate is a medium for cell culture or a buffer for cell homogenates.

8. Kit as claimed in claims 6 and 7, also provided with mixing systems to facilitate dissolution of the agonist, and/or thermostatic means to keep the temperature of the culture system and/or homogenization buffer in optimum conditions during the test.

9. Method for diagnosis of Huntington's Disease on a cell's sample *in-vitro*, **characterised by** assessing the receptor density of A_{2A} receptors in said cells, with respect to reference cells not affected by Huntington's Disease.

10. Method for the in-vitro diagnosis of neurodegenerative diseases caused by genetic mutations **characterised by** increased repetitions of the CAG triplet, said method being **characterised by** using, as a diagnostic markers the increase in cellular production of cyclic AMP following to treatment of said cells with A2A agonist compounds, or the increase of receptor density of A2A receptors.

11. Method according to claim 10, wherein said increase in cellular production of cyclic AMP or increase in A2A receptor density is assessed on cells'samples, with respect to corresponding reference cells not affected by said neurodegenerative disease.

12. Use of A_{2A} antagonist compounds for the preparation of a medicament for the treatment and/or prevention of Huntington's Disease.

## Patentansprüche

1. Methode für die Diagnose der Huntington-Erkrankung mithilfe einer *In-vitro-*Zellprobe, **dadurch gekennzeichnet, dass** genannte Zellen mit einer A_{2A} Agonist-Verbindung behandelt werden und danach bewertet wird, welche Menge an zyklischem AMP von den genannten Zellen produziert wird.

2. Verfahren gemäß Anspruch 1, umfassend die folgenden Schritte:
a- Bewertung mithilfe von gesunden Referenzzellen in Bezug auf den Anstieg der zellulären Produktion von zyklischem AMP, hervorgerufen durch Behandlung dieser Zellen mit A_{2A} Agonist-Verbindung;
b- Wiederholen von Schritt a mit den zu analysierenden Zellen;
c- Vergleichen des in Schritt a erhaltenen Wertes mit dem Wert in Schritt b.

3. Methode gemäß den Ansprüchen 1 und 2, durchgeführt in einer Pufferlösung mit einem pH-Wert im Bereich von 6,5 bis 8 und einer Temperatur im Bereich von 30°C bis 38°C.

4. Methode gemäß den Ansprüchen 1 bis 3, wobei es sich bei dem verwendeten A_{2A} -Agonisten um N-ethylcarboxamido-adenosin (NECA) handelt.

5. Methode gemäß den Ansprüchen 1 bis 4, wobei der A_{2A} -Agonist in einem Konzentrationsbereich von 10 nM bis 300 nM verwendet wird.

6. Kit für die Diagnose auf Huntington-Erkrankung, bestehend aus:
(i) ein Substrat, um eine Zellprobe im Vitalzustand zu halten
(ii) eine entsprechende Menge an A_{2A} Agonist-Verbindung
(iii) ein System für die Bestimmung von zyklischem AMP.

7. Kit gemäß Anspruch 6, wobei es sich bei dem genannten Substrat um ein Medium für Zellkultur oder einen Puffer für Zellhomogenate handelt.

8. Kit gemäß den Ansprüchen 6 und 7, beinhaltend Mischsysteme, um die Auflösung des Agonisten zu erleichtern und/oder thermostatische Vorrichtungen, um die Temperatur des Kultursystems und/oder des Homogenisationspuffers während des Tests im Optimalbereich zu halten.

9. Methode für die Diagnose der Huntington-Erkrankung mithilfe einer *In-vitro-*Zellprobe, **dadurch gekennzeichnet, dass** die Rezeptordichte des A_{2A} -Rezeptors in genannten Zellen unter Bezug auf die Referenzzellen, die nicht von der Huntington-Erkrankung betroffen sind, bewertet wird.

10. Methode für die *In*-*vitro*-Diagnose neurodegenerativer Erkrankungen verursacht durch genetische Mutationen, **gekennzeichnet durch** häufigere Wiederholungen des CAG-Triplets, wobei die genannte Methode **dadurch gekennzeichnet ist**, dass sie im Anschluss an die Behandlung der genannten Zellen mit A2A Agonist-Verbindungen als diagnostischen Marker den Anstieg der Zellproduktion an zyklischem AMP verwendet oder die Zunahme der Rezeptordichte des A2A-Rezeptors.

11. Methode gemäß Anspruch 10, wobei der Anstieg in der Zellproduktion von zyklischem AMP oder die Zunahme der A2A-Rezeptordichte mithilfe von Zellproben bewertet wird unter Bezugnahme auf die entsprechenden Referenzzellen, die nicht von genannter neurodegenerativer Erkrankung betroffen sind.

12. Verwendung von A_{2A} -Antagonist-Verbindungen für die Herstellung eines Medikaments zur Behandlung und/oder Prävention der Huntington-Krankheit.

## Revendications

1. Méthode de diagnostic de la maladie de Huntington sur un échantillon de cellules *in vitro*, **caractérisé par** le traitement desdites cellules avec un composé agoniste A_{2A}, suivi par l'évaluation de la quantité d'AMP cyclique produite par lesdites cellules.

2. Méthode selon la revendication 1 comprenant les étapes suivantes consistant à :
a- évaluer, sur des cellules de référence saines, l'augmentation de la production cellulaire d'AMP cyclique provoquée par le traitement de ces cellules avec un composé agoniste A_{2A} ;
b- répéter l'étape a sur les cellules à analyser ;
c- comparer la valeur obtenue à l'étape a avec celle obtenue à l'étape b.

3. Méthode selon la revendication 1 et la revendication 2, mise en oeuvre dans une solution tampon, à un pH appartenant à l'intervalle de 6,5 à 8, à une température appartenant à l'intervalle de 30 ° à 38 °C.

4. Méthode selon les revendications 1 à 3, dans laquelle l'agoniste A_{2A} utilisé est la N-éthylcarboxamido-adénosine (NECA).

5. Méthode selon les revendications 1 à 4, dans laquelle l'agoniste A_{2A} est utilisé à une concentration appartenant à l'intervalle de 10 nM à 300 nM.

6. Kit pour le diagnostic de la maladie de Huntington, composé de :
(i) un substrat pour maintenir un échantillon de cellules dans des conditions viables
(ii) une quantité appropriée d'un composé agoniste A_{2A}
(iii) un système pour la détermination de l'AMP cyclique.

7. Kit selon la revendication 6, dans lequel ledit substrat est un milieu de culture cellulaire ou un tampon pour homogénats cellulaires.

8. Kit selon les revendications 6 et 7, comprenant en outre des systèmes de mélangeage pour faciliter la dissolution de l'agoniste, et/ou des moyens thermostatiques pour maintenir la température du système de culture et/ou du tampon d'homogénéisation dans des conditions optimales pendant le test.

9. Méthode de diagnostic de la maladie de Huntington sur un échantillon de cellules *in vitro,* **caractérisée par** l'évaluation de la densité de récepteur des récepteurs A_{2A} dans lesdites cellules, par rapport à des cellules de référence non touchées par la maladie de Huntington.

10. Méthode de diagnostic *in vitro* de maladies de neurodégénérescence provoquées par des mutations génétiques **caractérisées par** une plus grande répétition du triplet CAG, ladite méthode étant **caractérisée par** l'utilisation, comme marqueurs de diagnostic, de l'augmentation de la production d'AMP cyclique cellulaire après traitement desdites cellules avec des composés agonistes A_{2A}, ou l'augmentation de la densité de récepteur des récepteurs A_{2A}.

11. Méthode selon la revendication 10, dans laquelle ladite augmentation de la production cellulaire d'AMP cyclique ou l'augmentation de la densité des récepteurs A_{2A} est évaluée sur des échantillons de cellules, par rapport à des cellules de référence correspondantes non touchées par ladite maladie de neurodégénérescence.

12. Utilisation de composés antagonistes A_{2A} pour la préparation d'un médicament pour le traitement et/ou la prévention de la maladie de Huntington.
